# EUROPEAN PATENT APPLICATION

(11) **EP 2 135 544 A1**
(43) Date of publication of application: **23.12.2009**
(21) Application number: 09007157.2
(22) Date of filing: 28.05.2009
(51) Int. Cl.: A61B 1/04

(54) **Processor device and method for displaying image**

(30) Priority: 19.06.2008 JP 2008160454
(71) Applicant: FUJIFILM Corporation, Minato-ku Tokyo (JP)
(72) Inventor: Hirano, Takeshi, Saitama-shi, Saitama (JP)
(74) Representative: Klunker . Schmitt-Nilson . Hirsch

(57) **Abstract**

A processor device (11) is connected to an electronic endoscope (10) having a CCD (40). The processor device produces an observation image (76) from an image signal of the electronic endoscope. In image quality adjustment, a menu screen (77) is generated based on an external command. Superimposing the menu screen on the observation image produces a menu composite image (78). When the menu screen covers an attention area (82) of the observation image in a standard image composition state, the observation image is shifted in the menu composite image. In the standard image composition state, the center of the observation image is positioned at the center of the menu composite image, and the menu screen is positioned at an end of the menu composite image.

## Description

### BACKGROUND OF THE INVENTION

### 1. Field of the Invention

The present invention relates to a processor device of an endoscope system, and a method for displaying an observation image and a menu screen.

### 2. Description Related to the Prior Art

In recent years, endoscope systems are widely used in medical examinations. The endoscope system is constituted of an electronic endoscope with a solid-state image sensor and a processor device to which the electronic endoscope is detachably connected. In the medical examination, a flexible insert section of the electronic endoscope is inserted into a human body cavity, and the solid-state image sensor provided at a distal portion of the insert section captures images of an internal body site. The processor device receives an image signal from the solid-state image sensor, and applies image processing thereto to generate observation images. The observation images are displayed on a monitor of the processor device.

In the processor device, a menu screen is generally displayed on the monitor for the purpose of reducing the size of an operation section and the number of operation buttons. Especially, displaying the menu screen that has image adjustment-related items such as contrast, chromaticity, and edge enhancement on the monitor offers practical convenience because an operator (doctor) can make system setting changes while watching the observation images.

When the menu screen and the observation image are displayed on the screen together, the menu screen occasionally overlaps the observation image and makes an attention area of the observation image invisible. Accordingly, Japanese Patent Laid-Open Publication No. 2005-110798 discloses to change the aspect ratio of the observation image, for example, from 16:9 to 4:3, and display the menu screen in space created by the aspect ratio change.

Changing the aspect ratio of the observation image, however, horizontally or vertically deforms the observation image, and brings a feeling of strangeness to the operator. The above publication uses a special monitor with an aspect ratio of 16: 9. On a conventional monitor with an aspect ratio of 4:3, however, changing the aspect ratio of the observation image cannot help shrinking the observation image to some extent, and consequently degrades resolution of the observation image.

### SUMMARY OF THE INVENTION

An object of the present invention is to provide a processor device and an image display method that can appropriately display an observation image and a menu screen without changing the aspect ratio of the observation image and degrading resolution thereof.

To achieve the above and other objects, a processor device according to the present invention is constituted of a signal processing circuit for producing an observation image from an image signal of the electronic endoscope, a menu screen generator for generating a menu screen based on an external command, an image composition circuit for making a menu composite image out of the observation image and the menu screen, and a monitor for displaying the menu composite image. The image composition circuit produces the menu composite image in such a manner that an attention area of the observation image is made visible without being covered with the menu screen.

When the menu screen covers the attention area in a standard image composition state, the image composition circuit shifts one of the observation image and the menu screen in the menu composite image. In the standard image composition state, the center of the observation image is positioned at the center of the menu composite image, and the menu screen is positioned at an end of the menu composite image.

The observation image includes a pickup image captured by the electronic endoscope and a rectangular mask area surrounding the pickup image. The pickup image is circular or rectangular. The menu composite image is rectangular of larger size, and the menu screen is rectangular of smaller size. In the standard image composition state, a corner of the menu screen coincides with a corner of the menu composite image.

It is preferable that the image composition circuit shift the observation image in the menu composite image so that the attention area is away from the menu screen.

It is known by experience that the attention area often exists in the middle of the pickup image. The attention area may be extracted by image analysis of the pickup image. The attention area may be a blood vessel intensive area. In extracting the blood vessel intensive area, the attention area extraction circuit first increases red-color in the pickup image and then extracts an area with high red-color concentration.

The attention area may be extracted by pattern matching using a predetermined pattern. Otherwise, the attention area extraction circuit may calculate an image feature amount that distinguishes a specific image from block to block of the pickup image, and extract the specific image as the attention area based on the image feature amount.

In a preferred embodiment of the present invention, when the menu screen covers the attention area, an operator shifts the observation image in the menu composite image by external operation with watching the menu composite image on the monitor.

An image display method comprises an observation image producing step, a menu screen generating step, a menu composite image producing step, an image shifting step, and a menu composite image displaying step. In the observation image producing step, an observation image is produced from an image signal of an electronic endoscope. In the menu screen generating step, a menu screen is generated based on an external command. In the menu composite image producing step, a menu composite image is produced from the observation image and the menu screen. In the image shifting step, the observation image or the menu screen is shifted in the menu composite image when the menu screen covers an attention area of the observation image. In the menu composite image display step, the menu composite image after the shift is displayed on a monitor.

According to the processor device and the image display method of the present invention, when the menu screen is displayed on the observation image, the observation image or the menu screen is shifted so that the menu screen does not cover the attention area. Accordingly, it is possible to favorably display both of the observation image and the menu screen without changing the aspect ratio of the observation image and degrading the resolution thereof.

### BRIEF DESCRIPTION OF THE DRAWINGS

For more complete understanding of the present invention, and the advantage thereof, reference is now made to the following descriptions taken in conjunction with the accompanying drawings, in which:
Fig. 1 is an explanatory view of an endoscope system;
Fig. 2 is a front view of a distal portion of an electronic endoscope;
Fig. 3 is a block diagram of an endoscope system according to a first embodiment;
Fig. 4 is an explanatory view showing observation image producing procedure;
Fig. 5 is an explanatory view showing the process of superimposing a menu screen on the observation image;
Fig. 6 is a flowchart of display process according to a second embodiment;
Fig. 7 is a block diagram of an endoscope system according to a third embodiment;
Fig. 8 is an explanatory view of menu composition process according to the third embodiment; and
Fig. 9 is an explanatory view of menu composition process according to a fourth embodiment.

### DESCRIPTION OF THE PREFERRED EMBODIMENTS

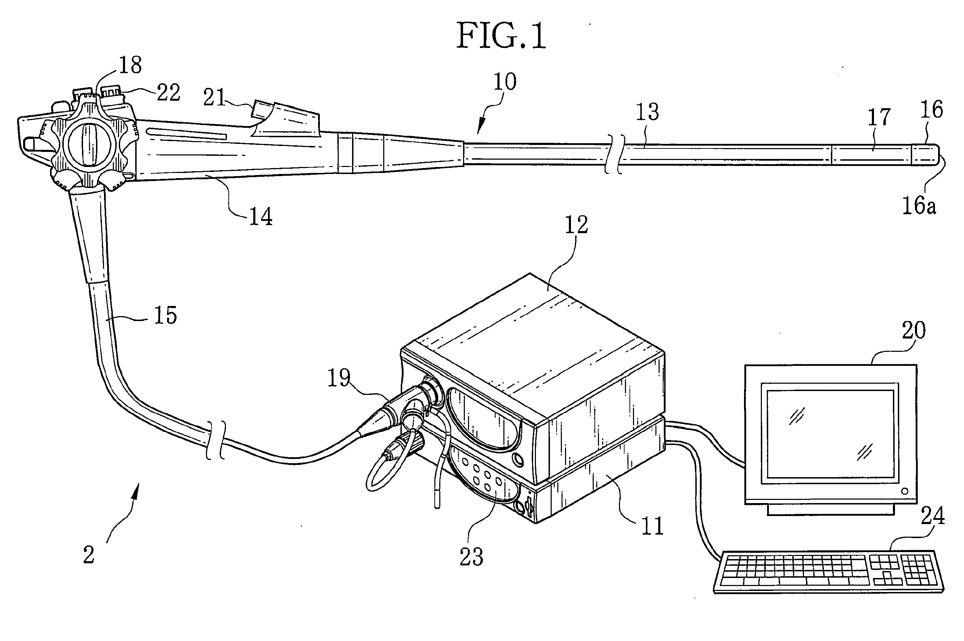

In Fig. 1, an endoscope system 2 is constituted of an electronic endoscope 10, a processor device 11, and a light source device 12. The electronic endoscope 10 is provided with a flexible insert section 13 to be introduced into a human body cavity, a handling section 14 coupled to a base end of the insert section 13, and a universal cord 15 connected to the processor device 11 and the light source device 12.

At the tip of the insert section 13 is provided a distal portion 16 that contains a CCD image sensor 40 (hereinafter referred to as CCD). Behind the distal portion 16, there is provided a bending portion 17 that consists of a number of linked ring-like segments. Operating an angle knob 18 on the handling section 14 pulls and pushes wires extending in the insert section 13 to bend the bending portion 17 from side to side and up and down. Thus, the distal portion 16 is aimed at a desired direction inside the human body cavity.

To an end of the universal cord 15, a multi-connector 19 is attached. The electronic endoscope 10 is detachably connected to the processor device 11 and the light source device 12 via the connector 19.

The processor device 11 receives an image signal from the CCD 40, and subjects the image signal to various kinds of signal processing. The processed image signal is displayed on a monitor 20, which is connected to the processor device 11 with a wire, as pickup images. The processor device 11 is electrically connected to the light source device 12, and controls the entire operation of the endoscope system 2.

The handling section 14 of the electronic endoscope 10 is provided with a medical instrument insertion port 21, the angle knob 18, and operation buttons including an airing/watering button 22.

A front panel 23 is provided in a front face of the processor device 11. The front panel 23 has a menu screen display button that is operated to display a desired menu screen on the monitor 20 and setting buttons for changing image processing conditions (contrast, chromaticity, edge enhancement, and the like). In addition to the monitor 20, a keyboard 24 is connected to the processor device 11 with a wire for the purpose of typing patient data (a patient's ID number, a patient's name, sex, and a birthday) and the like.

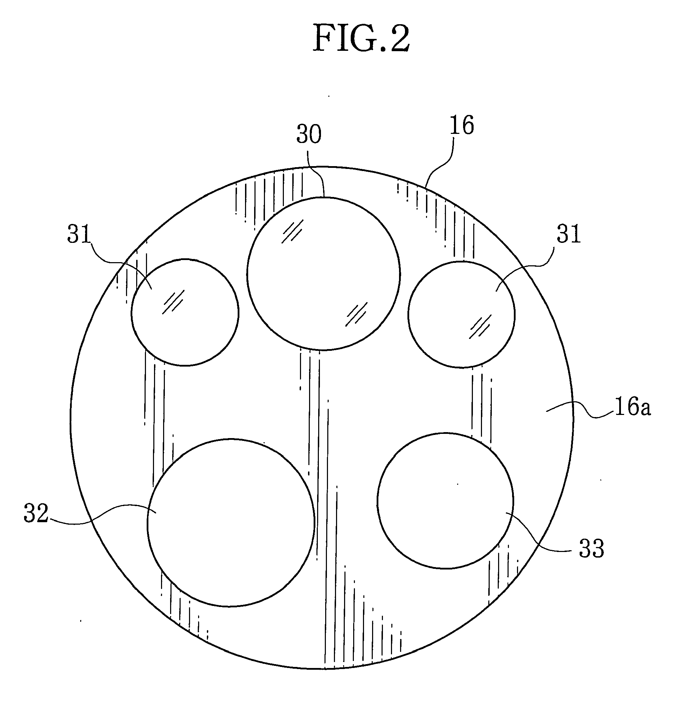

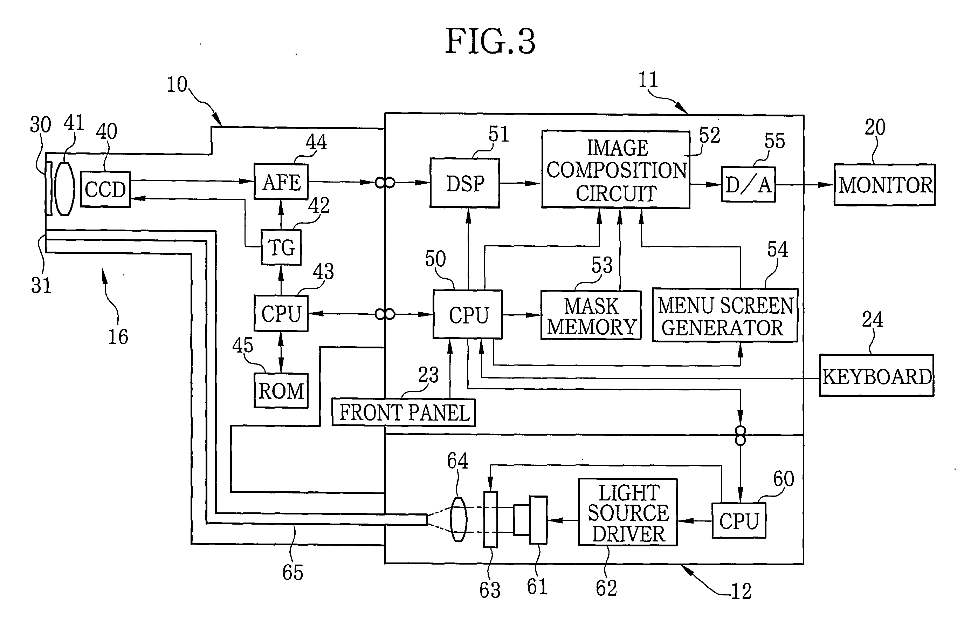

As shown in Fig. 2, a front face 16a of the distal portion 16 is provided with an image capturing window 30, lighting windows 31, a medical instrument outlet 32, and an airing/watering nozzle 33. The image capturing window 30 is disposed in the upper middle of the front face 16a. The two lighting windows 31 are symmetric with respect to the image capturing window 30. Through the lighting windows 31, illumination light, which is led from the light source device 12 via a light guide 65 (refer to Fig. 3), is incident on a target body part in the human body cavity. The medical instrument outlet 32 is coupled to the medical instrument insertion port 21 through a not-illustrated channel extending in the insert section 13. A medical instrument with a pair of forceps, an injection needle, a diathermy knife, or the like at its tip is inserted into the medical instrument insertion port 21 in order to protrude the tip of the instrument from the medical instrument outlet 32 in the human body cavity. Water or air supplied by a not-illustrated air/water reservoir contained in the light source device 12 is sprayed through the watering/airing nozzle 33 on the image capturing window 30 or the target body part in response to operation of the watering/airing button 21.

Referring to Fig. 3, the electronic endoscope 10 has the CCD 40, a timing generator (TG) 42, an analog front end processor (AFE) 44, a CPU 43, and a ROM 45. The CCD 40 is contained in the distal portion 16 of the electronic endoscope 10. The CCD 40 is disposed in the focal plane of an objective lens 41, which is disposed oppositely to the image capturing window 30. A light receiving surface of the CCD 40 is equipped with a color filter having a plurality of color segments (for example, primary-colors filter of Bayer arrangement).

The TG 42 generates drive pulses (clock pulses, vertical and horizontal scan pulses, a reset pulse, and the like) for the CCD 40 and synchronizing pulses for the AFE 44 based on control of the CPU 43. The CCD 40 that is driven by the drive pulses from the TG 42 performs photoelectric conversion on an optical image formed by the objective lens 41, and outputs the image signal.

The AFE 44 is constituted of a correlated double sampling circuit (CDS), a programmable gain amplifier (PGA), and an A/D converter (A/D). The CDS applies correlated double sampling processing to the image signal from the CCD 40 in order to remove reset noise and amplifier noise caused by the CCD 40. The PGA amplifies the image signal without noise by gain designated by the CPU 43. The A/D converts the amplified image signal into a digital image signal of a predetermined number of bits. The digital image signal outputted from the AFE 44 is inputted to the processor device 11 through the connector 19.

The CPU 43 communicates with a CPU 50 of the processor device 11 to control individual parts of the electronic endoscope 10. The CPU 43 is connected to the ROM 45 that stores identification data for identifying the model of the electronic endoscope 10. The CPU 43 reads the identification data from the ROM 45, and inputs the identification data to the CPU 50 of the processor device 11.

The processor device 11 includes the CPU 50, a digital signal processor (DSP) 51, an image composition circuit 52, a mask memory 53, a menu screen generator 54, and a D/A converter (D/A) 55. The CPU 50 controls individual parts of the processor device 11 and the whole of the endoscope system 2. The DSP 51 applies color interpolation, color separation, color balance adjustment, gamma correction, edge enhancement processing, and the like to the image signal of a single frame inputted from the AFE 44 of the electronic endoscope 10, and generates an original image.

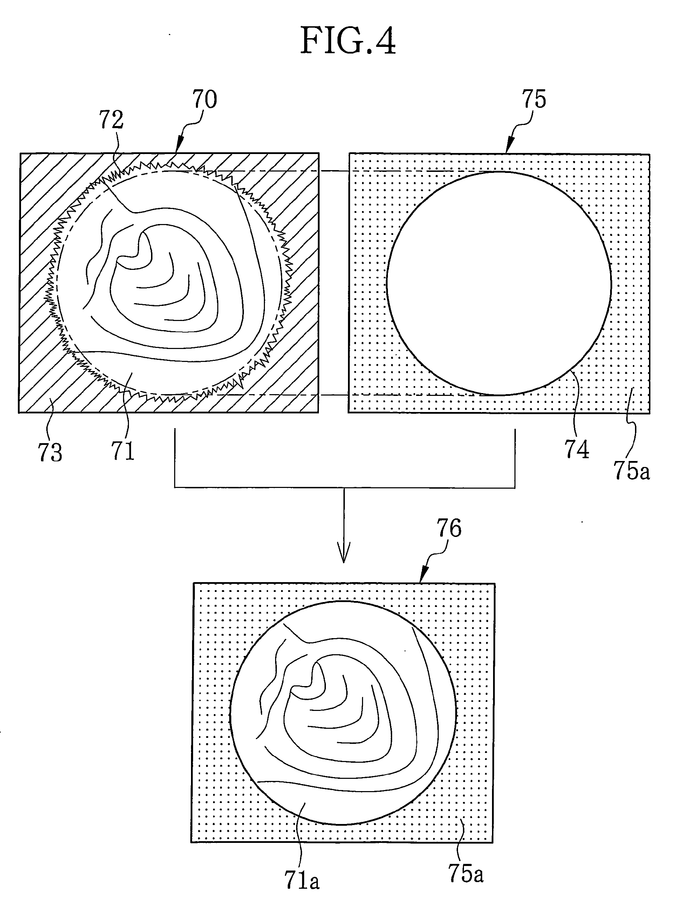

The image composition circuit 52 superimposes a mask image 75 stored on the mask memory 53 on the original image outputted from the DSP 51 according to control of the CPU 50. To be more precise, as shown in Fig. 4, the rectangular original image 70 includes a pickup image area 71 allocated within a circular serrated region 72 and a vignette area 73 allocated outside thereof. The serrated region 72 is formed by a lens barrel frame. The mask memory 53 stores the mask image 75 that has an opening 74 positioned in the middle and a color-filled mask area 75a set around the opening 74. The image composition circuit 52 superimposes the mask image 75 on the original image 70, and generates a rectangle observation image (mask composite image) 76 as shown in a lower part of Fig. 4. The observation image 76 consists of a round pickup image 71a and the mask area 75a surrounding the pickup image 71a.

Since the position and size of the serrated region 72 in the original image 70 varies with the model of the electronic endoscope 10, a plurality of mask images 75 having openings 74 in various sizes and shapes is prepared in the mask memory 53. The CPU 50 chooses the proper mask image 75 from the mask memory 53 based on the identification data of the electronic endoscope 10, and supplies the mask image 75 to the image composition circuit 52.

Before displaying a menu screen, the image composition circuit 52 outputs the observation image 76 to the D/A 55. The D/A 55 converts the observation image 76 into an analog image signal, and displays the observation image 76 on the monitor 20.

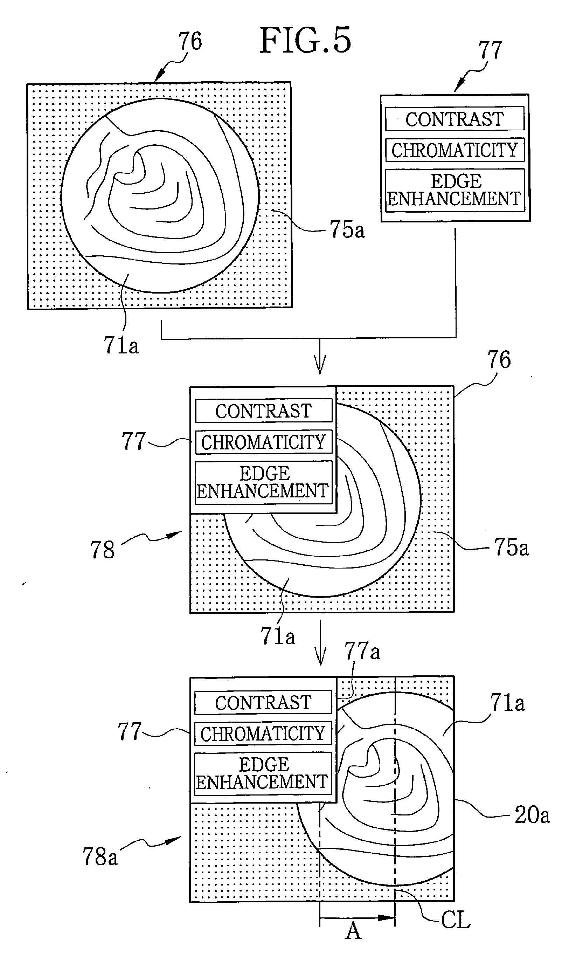

Upon inputting a menu screen display command signal from the front panel 23, the menu screen generator 54 generates a menu screen 77 based on control of the CPU 50. There is a plurality of menu screens 77 such as an image processing conditions setting screen for changing the image processing condition settings and a patient's data input screen for inputting patient's data. The menu screen generator 54 generates the chosen menu screen 77 in response to the menu screen display command signal, and inputs the menu screen 77 to the image composition circuit 52. The image composition circuit 52 superimposes the menu screen 77 on the observation image 76. In a standard image composition state, as shown in Fig. 5, the image composition circuit 52 superimposes the menu screen 77 on the observation image 76 so as to vertically align the upper left corner of the menu screen 77 with that of the observation image 76, and produces a menu composite image 78.

In capturing an image, in general, an attention area is positioned in the middle of the pickup image 71a. In this embodiment, it is assumed that the attention area exists in the middle of the pickup image 71a. If the menu screen 77 covers the middle of the pickup image 71a in the menu composite image 78 in the standard image composition state, the observation image 76 is automatically shifted to the right in the menu composite image 78 so as to make the middle of the pickup image 71a visible. Accordingly, it is generated a menu composite image 78a in which the attention area of the pickup image 71a is made visible. The image composition circuit 52 outputs the menu composite image 78a after the shift to the D/A 55. The D/A 55 converts the menu composite image 78a into an analog image signal, and outputs the image signal to the monitor 20.

As shown in a lower part of Fig. 5, it is preferable that the distance "A" of shifting the observation image 76 be determined in such a manner that the center line CL of the pickup image 71a coincides with the center line between a right edge 77a of the menu screen 77 and a right edge 20a of the menu composite image 78, that is, a right edge of a display area of the monitor 20. In this case, shifting the observation image 76 causes space on the left of the menu composite image 78a, and the mask area 75a is expanded to fill the space.

The light source device 12 is constituted of a CPU 60, a light source 61 such as a xenon lamp or a halogen lamp, a light source driver 62, an aperture stop mechanism 63, and a condenser lens 64. The CPU 60 communicates with the CPU 50 of the processor device 11, and controls the light source driver 62 and the aperture stop mechanism 63. The light source driver 62 drives the light source 61. The aperture stop mechanism 63, which is disposed on a light emission side of the light source 61, increases or decreases the amount of illumination light incident upon the condenser lens 64. The condenser lens 64 condenses the illumination light that has passed through the aperture stop mechanism 63, and leads the illumination light into an entry of the light guide 65. The light guide 65 extends from the base end of the electronic endoscope 10 to the distal portion 16, and is branched off in two exits in the distal portion 16. Each exit is connected to corresponding one of the two lighting windows 31 provided in the front face 16a of the distal portion 16.

Next, the operation of the endoscope system 2 will be described. In examining the inside of the human body cavity by using the endoscope system 2, the electronic endoscope 10, the processor device 11, the light source device 12, and the monitor 20 are turned on, and the insert section 13 of the electronic endoscope 10 is inserted into the human body cavity. While the illumination light from the light source device 12 illuminates the inside of the body cavity, the CCD 40 captures images of the target body part.

During the capture of the images, in the processor device 11, the original image 70 is inputted from the DSP 51 to the image composition circuit 52. The image composition circuit 52, as shown in Fig. 4, superimposes the mask image 75 on the original image 70 to generate the observation image 76. The observation image 76 is displayed on the monitor 20 through the D/A 55.

While the observation image 76 is displayed on the monitor 20, if the change of image processing conditions (contrast, chromaticity, edge enhancement, and the like) or the input of patient's data (patient's ID number, patient's name, sex, birthday, and the like) is desired, a predetermined operation button on the front panel 23 is pressed.

In response to the press of the predetermined operation button, the menu screen display command signal is inputted to the CPU 50. Receiving the menu screen display command signal, the CPU 50 makes the menu screen generator 54 generate the menu screen 77 and input the menu screen 77 to the image composition circuit 52. The image composition circuit 52, as shown in Fig. 5, superimposes the menu screen 77 on the observation image 76. When the menu screen 77 covers the middle of the pickup image 71a, the image composition circuit 52 automatically shifts the observation image 76 to make the middle of the pickup image 71 visible. Therefore, the menu composite image 78a is generated in a state of the attention area of the pickup image 71 being visible. The menu composite image 78a after the shift is displayed on the monitor 20 through the D/A 55. The menu composite image 78 in the standard image composition state is shown in Fig. 5 just for the sake of explanation, and is not actually displayed on the monitor 20. The menu composite image 78a after the shift is directly displayed on the monitor 20.

Accordingly, it is possible to choose a desired menu on the menu screen 77 and adjust image quality or the like with observing the attention area of the pickup image 71a. After completing the image quality adjustment, the menu screen 77 disappears and only the observation image 76 is displayed on the middle of the monitor 20 again in response to a press of the predetermined operation button on the front panel 23.

In the processor device 11 according to the present invention, as described above, when the menu screen 77 is displayed on the observation image 76, if the menu screen 77 covers the middle of the pickup image 71a, the observation image 76 is automatically shifted to make the attention area, existing in the middle of the pickup image 71a, visible. Thus, it is possible to carry out desired operation on the menu screen 77 with observing the attention area of the pickup image 71a. The attention area may not be the middle of the pickup image 71 but another part.

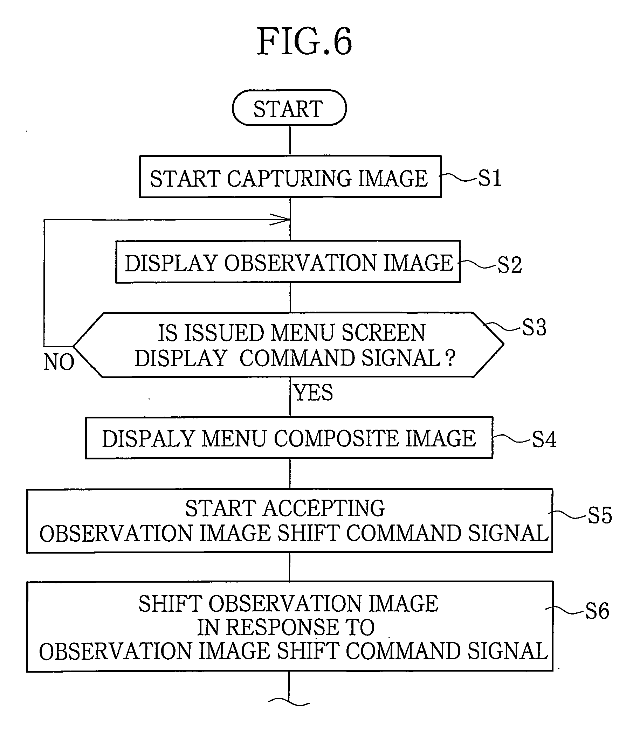

Next, a second embodiment of the present invention will be described with referring to Fig. 6. In a processor device of the second embodiment, when the menu screen 77 covers the attention area of the pickup image 71a, the observation image 76 is manually shifted based on an observation image shift command signal inputted from the keyboard 24, instead of automatically shifted in the menu composite image. The other configuration is the same as that of the first embodiment.

In the second embodiment, when the CCD 40 starts capturing the images (step S1), the observation image 76 is displayed on the monitor 20 (step S2) as with the first embodiment. While the observation image is displayed, if the predetermined operation button on the front panel 23 is operated to input the menu screen display command signal to the CPU 50 (YES of step S3), the image composition circuit 52 superimposes the menu screen 77 outputted from the menu screen generator 54 on the observation image 76. At this time, even if the menu screen 77 covers the middle of the pickup image 71a, the observation image 76 is not automatically shifted. The menu composite image 78 in the standard image composition state is displayed on the monitor 20 (step S4).

The CPU 50 accepts the observation image shift command signal from an operator (step S5). Pressing, for example, a right or left arrow key on the keyboard 24 can input the observation image shift command signal to the CPU 50. In response to pressing the right arrow key, the image composition circuit 52 shifts the observation image 76 in the right direction by a distance corresponding to the duration or the number of times the right arrow key has been pressed, as shown in the lower part of Fig. 5. Upon pressing the left arrow key, on the other hand, the image composition circuit 52 shifts the observation image 76 to the left (step S6).

According to this embodiment, when the menu screen 77 covers the attention area of the pickup image 71a, the observation image 76 can be shifted by the operation of the keyboard 24. Accordingly, the operator can watch the attention area of the pickup image 71a without being obstructed by the menu screen 77.

The observation image shift command signal for shifting the observation image 76 may be inputted from another operation section such as the front panel 23 instead of the keyboard 24. When the menu screen 77 is superimposed on the attention area of the pickup image 71, the observation image 76 may be first shifted automatically as described in the first embodiment, and then further shifted by manual operation.

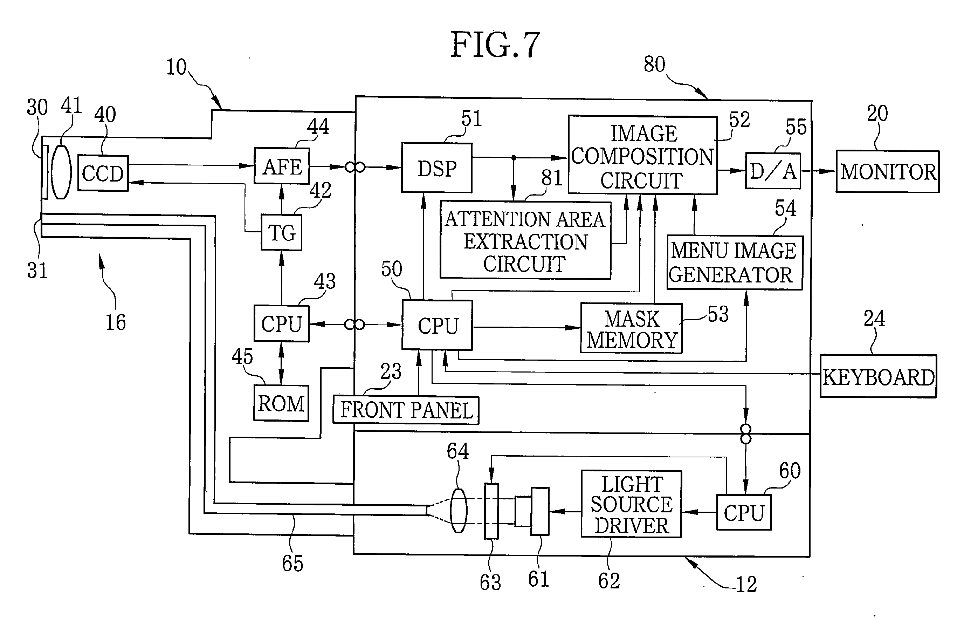

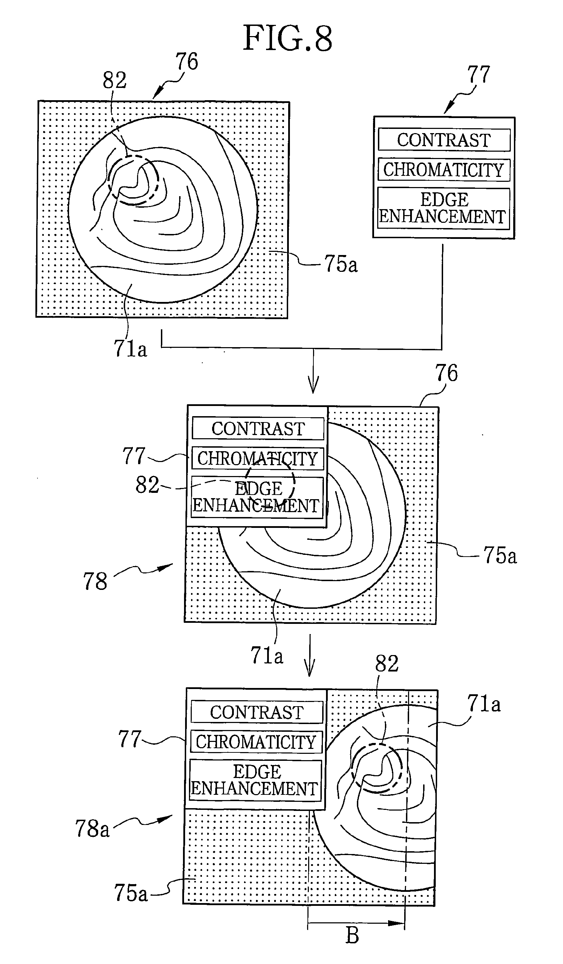

Next, a third embodiment of the present invention will be described with referring to Figs. 7 and 8. A processor device 80 according to the third embodiment automatically extracts the attention area of the pickup image 71 by image analysis, and automatically shifts the observation image 76 in the menu composite image so that the menu screen 77 does not cover the extracted attention area. The other configuration is the same as that of the first embodiment. The same reference numbers as Figs. 3 and 5 refer to identical or functionally similar components.

The processor device 80 is provided with an attention area extraction circuit 81. The attention area extraction circuit 81 takes out the original image 70 from the DSP 51, and applies blood vessel enhancement processing on the original image 70 to extract a blood vessel concentration area. The attention area extraction circuit 81 determines the blood vessel intensive area as the attention area. The blood vessel enhancement processing is disclosed in, for example, Japanese Patent Laid-Open Publication No. 2003-93342. The attention area extraction circuit 81, adopting this technology, first generates a differential signal of a color signal (for example, green signal) other than a red signal being a main color signal of a blood vessel, and then amplifies the red signal based on the differential signal to enhance the blood vessel. Then, the attention area extraction circuit 81 extracts an area of high red concentration from the original image 70 under red-color enhancement, and sets the extracted area as the attention area 82.

The attention area 82 extracted by the attention area extraction circuit 81 is inputted to the image composition circuit 52. The image composition circuit 52 shifts the observation image 76 in the menu composite image so that the menu screen 77 does not cover the attention area. Taking a case where the attention area 82 is set at the upper left of the pickup image 71a as an example, as shown in Fig. 8, when the menu screen 77 is superimposed on the upper left of the observation image 76, the menu screen 77 covers the attention area 82 and makes the attention area 82 invisible. Thus, as shown in a lower part of Fig. 8, the observation image 76 is shifted by a distance "B" in the right direction according to the degree of overlapping the menu screen 77 in order to make the attention area 82 visible. The pickup image 71a displayed on the monitor 20 is not subjected to the blood vessel enhancement processing, but may be subjected thereto.

In the third embodiment, as described above, the blood vessel intensive area is automatically extracted as the attention area 82 from the pickup image 71a by the blood vessel enhancement processing, and automatically shifts the observation image 76 so that the menu screen 77 does not obstruct the attention area 82. Therefore, it is possible to watch the attention area 82 in the menu composite image 78 nevertheless the attention area 82 is not in the middle of the pickup image 71.

As another method for extracting the blood vessel intensive area, the pickup image 71a is divided into a plurality of small blocks, and the image feature amount of a blood vessel pattern is calculated from block to block. In this method, the block having the largest image feature amount is decided to be the blood vessel intensive area. As the types of the image feature amount of the blood vessel pattern, there are "distribution of direction of blood vessel edges", "the number and positional relation of blood vessel branches", and the like. Since the blood vessel branch has the so-called fractal structure, a fractal dimension value of the blood vessel edge (a value that quantifies the complexity of the pattern) may be set as the image feature amount. In this case, the fractal dimension value is calculated from block to block, and the block having the largest fractal dimension value is decided to be the blood vessel intensive area.

Since the blood vessel intensive area is an important observed area for diagnosing the focus of disease and finding a lesion, it is preferable to set the blood vessel intensive area as the attention area. However, an area of a lesion or the like detected by pattern recognition may be extracted as the attention area 82, instead of the blood vessel intensive area. For the pattern recognition, it is available a commonly known face detection technology adopted in a digital camera (refer to, for example, Japanese Patent Laid-Open Publication Nos. 2005-284203 and. 2005-156967 and US Patent Application Publication No. 2005/0219395 (corresponding to Japanese Patent Laid-Open Publication No. 2005-286940)). Specifically, the pattern of a lesion or the like is prepared as a template, and the agreement degree with the template in shape and color is detected on a predetermined search-area basis in the pickup image 71a. Detection is carried out on the whole pickup image 71a with varying the size and angle of the search-area, and a part having the highest agreement degree is decided to be the attention area 82.

In the first to third embodiments, the observation image 76 is shifted to the right so that the menu screen 77 does not cover the attention area 82. The observation image 76, however, is shifted upwardly, downwardly, or diagonally in accordance with the position of the menu screen 77. The shift amount of the observation image 76 is changed as needed in accordance with the model of the electronic endoscope 10, in other words, the size and shape of the opening 74.

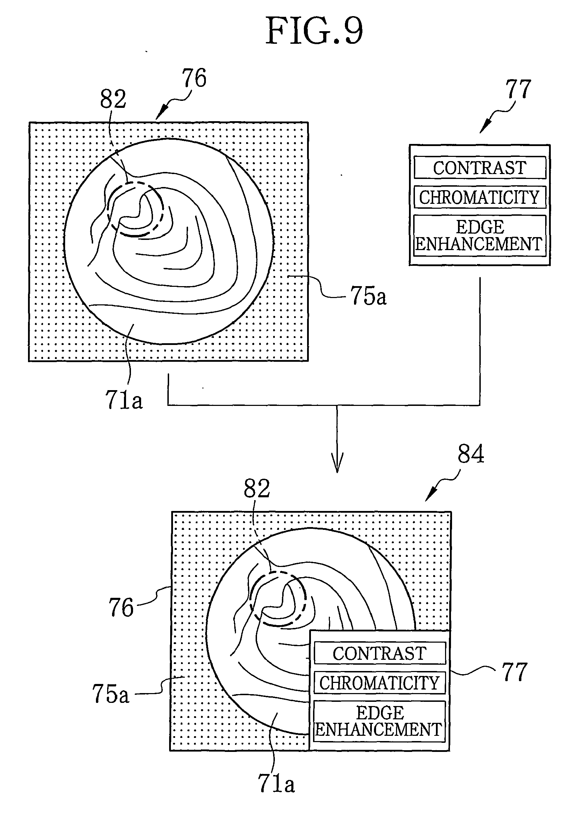

Next, a fourth embodiment of the present invention will be described with referring to Fig. 9. In the fourth embodiment, the image composition circuit 52 obtains positional information of the middle part of the pickup image 71a or the attention area 82 extracted by the attention area extraction circuit 81 in advance. The image composition circuit 52 automatically varies the position of displaying the menu screen 77 so that the menu screen 77 does not obstruct the attention area 82.

Taking a case where the attention area 82 of the pickup image 71a exists at the upper left of the observation image 76 as an example, the menu screen 77 would cover the attention area 82 in the menu composite image 78 in the standard image composition state. In this case, the image composition circuit 52 superimposes the menu screen 77 on the observation image 76 in such a manner as to vertically align the lower right corner of the menu screen 77 with that of the observation image 76 to produce the menu composite image 84. Thus, the menu screen 77 does not cover the attention area 82 of the pickup image 71a.

The display position of the menu screen 77 is not limited to the lower right or the upper left of the observation image 76 but is properly changeable. The size of the menu screen 77 may be properly reduced without making letters indicating menu items hard to see.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. A processor device (11) for an electronic endoscope (10) comprising:
a signal processing circuit (51) for producing an observation image (76) from an image signal of said electronic endoscope;
a menu screen generator (54) for generating a menu screen (77) based on an external command;
an image composition circuit (52) for superimposing said menu screen on said observation image to produce a menu composite image (78a) in such a manner that an attention area (82) of said observation image is made visible without being covered with said menu screen; and
a monitor (20) for displaying said menu composite image.

2. The processor device (11) as recited in claim 1, wherein when said menu screen (77) covers said attention area (82) in a standard image composition state, said image composition circuit (52) shifts one of said observation image (76) and said menu screen in said menu composite image (78), wherein in said standard image composition state, the center of said observation image is positioned at the center of said menu composite image, and said menu screen is positioned at an end of said menu composite image.

3. The processor device (11) as recited in claim 2, wherein said observation image (76) includes a pickup image (71a) captured by said electronic endoscope (10) and a rectangular mask area (75a) surrounding said pickup image.

4. The processor device (11) as recited in claim 3, wherein said menu screen (77) is rectangle, and said menu screen is fitted in a corner of said menu composite image (78) in said standard image composition state.

5. The processor device (11) as recited in claim 4, wherein said image composition circuit (52) shifts said observation image (76) in said menu composite image (78) so that said attention area (82) is away from said menu screen (77).

6. The processor device (11) as recited in claim 5, wherein said attention area (82) exists in the middle of said pickup image (71a).

7. The processor device (11) as recited in claim 4, further comprising:
an attention area extraction circuit (81) for extracting said attention area (82) by image analysis of said pickup image (71a).

8. The processor device (11) as recited in claim 7, wherein said attention area extraction circuit (81) increases red-color in said pickup image (71a) and then extracts a blood vessel intensive area with high red-color concentration as said attention area (82).

9. The processor device (11) as recited in claim 7, wherein said attention area extraction circuit (81) extracts said attention area (82) by pattern matching using a predetermined pattern.

10. The processor device (11) as recited in claim 7, wherein said attention area extraction circuit (81) calculates an image feature amount for distinguishing a specific image from block to block of said pickup image (71a), and extracts said specific image as said attention area (82) based on said image feature amount.

11. A processor device (11) for an electronic endoscope (10) comprising:
a signal processing circuit (51) for producing an observation image (76) from an image signal of said electronic endoscope;
a menu screen generator (54) for generating a menu screen (77) based on an external command;
an image composition circuit (52) for superimposing said menu screen on said observation image to produce a menu composite image (78);
a monitor (20) for displaying said menu composite image; and
a control circuit (50, 52) for shifting said observation image in said menu composite image based on an external command.

12. The processor device (11) as recited in claim 11, wherein
said menu screen (77) is rectangular;
the center of said observation image (76) is positioned at the center of said menu composite image (78), and said menu screen is positioned at an end of said menu composite image in a standard image composition state; and
said control circuit (50, 52) shifts said observation image from a position of said standard image composition state.

13. A method for displaying an image for a processor device (11) connected to an electronic endoscope (10), said method comprising the steps of:
producing an observation image (76) from an image signal of said electronic endoscope;
generating a menu screen (77) based on an external command;
superimposing said menu screen on said observation image to produce a menu composite image(78);
shifting said observation image or said menu screen in said menu composite image when said menu screen covers an attention area (82) of said observation image; and
displaying said menu composite image on a monitor (20).
